# FASCICULE DE BREVET EUROPEEN

(11) **EP 0 874 847 B1**
(45) Date de publication et mention de la délivrance du brevet: **19.03.2003**
(21) Numéro de dépôt: 96941088.5
(22) Date de dépôt: 29.11.1996
(51) Int. Cl.: C07D 473/16, C07D 473/40, C07D 473/32, A61K 31/52

(54) **NOUVEAUX DERIVES DE PURINE POSSEDANT NOTAMMENT DES PROPRIETES ANTI-PROLIFERATIVES ET LEURS APPLICATIONS BIOLOGIQUES**
PURINEDERIVATE MIT ANTIPOLIFERATIVER WIRKUNG UND IHRE BIOLOGISCHEN EIGENSCHAFTEN
NOVEL PURINE DERIVATIVES HAVING, IN PARTICULAR, ANTIPROLIFERATIVE PROPERTIES, AND BIOLOGICAL USES THEREOF

(30) Priorité: 01.12.1995 FR 9514237
(43) Date de publication de la demande: 04.11.1998
(60) Demande divisionnaire: 02293256.0
(73) Titulaire: CENTRE NATIONAL DE LA RECHERCHE SCIENTIFIQUE, 75016 Paris Cédex (FR); Institut de Botanique Expérimentale, 165 02 Praha 6 (CZ)
(72) Inventeur: MEIJER, Laurent, F-29650 Roscoff (FR); BISAGNI, Emile, F-91400 Orsay (FR); LEGRAVEREND, Michel, F-92160 Antony (FR); STRNAD, Miroslav, 779 00 OLOMOUC (CZ)
(74) Mandataire: Peaucelle, Chantal
(86) Numéro de dépôt international: FR9601905
(87) Numéro de publication internationale: WO97020842

(56) Documents cités:
- CHEMICAL ABSTRACTS, vol. 104, no. 19, 12 Mai 1986 Columbus, Ohio, US; abstract no. 164159a, page 274; colonne l; XP002009710 & PHYTOCHEMISTRY, vol. 25, no. 2, 1986, pages 303-310,
- CHEMICAL ABSTRACTS, vol. 115, no. 23, 9 Décembre 1991 Columbus, Ohio, US; abstract no. 252157f, page 471; colonne l; XP002009711 & PHYTOCHEMISTRY, vol. 30, no. 8, 1991, pages 2477-2486,
- CHEMICAL ABSTRACTS, vol. 117, no. 5, 3 Août 1992 Columbus, Ohio, US; abstract no. 44644b, page 534; colonne l; XP002009712 & J. PLANT GROWTH REGUL., vol. 10, no. 4, 1991, pages 179-185,
- EUROPEAN JOURNAL OF BIOCHEMISTRY, vol. 224, no. 2, 1 Septembre 1994, pages 771-786, XP002009709 JAROSLAV VESELY ET AL: cité dans la demande

## Description

L'invention a pour objet de nouveaux dérivés de purine possédant des propriétés anti-prolifératives et leurs applications biologiques.

Elle vise en particulier des dérivés de purine dotés d'un effet inhibiteur vis-à-vis de protéines kinases cycline-dépendantes, ou cdk en abrégé.

L'étude des mécanismes moléculaires qui contrôlent le cycle cellulaire a permis de mettre en évidence le rôle régulateur des cdk. Ces protéines sont constituées d'au moins deux sous-unités, une sous-unité catalytique (dont cdc2 est le prototype) et une sous-unité régulatrice (cycline). Huit cdk ont été décrites, cdk1 (=cdc2), cdk2-cdk8.

A l'exception de cdk3, pour laquelle aucune cycline associée n'est connue, ces cdk sont régulées par association transitoire avec un membre de la famille des cyclines : cycline A (cdc2, cdk2), cycline B1-B3 (cdc2), cycline C (cdk8), cycline D1-D3 (cdk2-cdk4-cdk5-cdk6), cycline E (cdk2), cycline H (cdk7).

Chacun de ces complexes est impliqué dans une phase du cycle cellulaire. L'activité des cdk est régulée par modification post-traductionnelle, par associations transitoires avec d'autres protéines et par modification de leur localisation intracellulaire. Les régulateurs des cdk comprennent des activateurs (cyclines, cdk7/cycline H, phosphatases cdc25), les sous-unités p9^{CKS} et p15^{cdk-BP} et les protéines inhibitrices (p16^{INK4A}, p15^{INK4B}, p21^{Cip1}, p18, p27^{Kip1}).

Parallèlement aux recherches purement fondamentales sur les mécanismes régulateurs de la division cellulaire, l'importance des dérégulations des kinases cyclines-dépendantes dans le développement des tumeurs humaines a été démontrée par de nombreuses études. Ainsi, ont été observées la surexpression de cyclines D et E dans de nombreuses tumeurs, la surexpression de cdc2, les propriétés oncogéniques des cyclines D et A, l'expression temporelle anormale de kinases cyclines-dépendantes, la dérégulation majeure des inhibiteurs protéiques (mutations, délétions).

Les régulateurs du cycle de division cellulaire font l'objet de très nombreuses études cliniques (utilisation en tant que marqueurs indicatifs pour le traitement).

Ces résultats encouragent très fortement les efforts de compréhension détaillée des mécanismes régulateurs du cycle cellulaire. Ils conduisent également à rechercher, par criblage, des molécules inhibitrices de kinases cyclines-dépendantes.

Plusieurs inhibiteurs de kinases ont été décrits, comme la butyrolactone, le flavopiridol et la 2(2-hydroxyéthylamino)-6-benzylamino-9-méthylpurine, appelée olomoucine. Les travaux concernant l'olomoucine sont rapportés par Vesely et al. dans l'article portant la référence (1) dans la liste des références bibliographiques donnée en fin de description.

Cet inhibiteur de cdc2, de grande efficacité (son IC₅₀ est de 7 µM) et très sélectif (plus de 35 kinases ont été testées), répond à la formule :

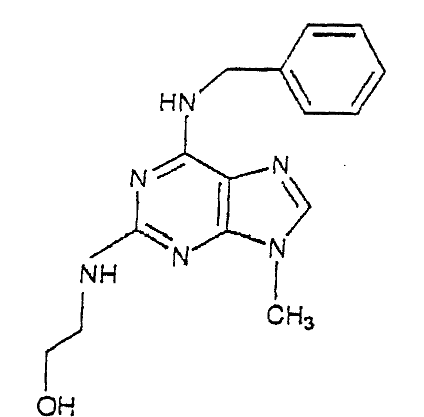

Les travaux des inventeurs dans ce domaine les ont conduits à élaborer de nouvelles molécules particulièrement intéressantes, inhibant cdc2 à doses faibles, tout en conservant la spécificité enzymatique de l'olomoucine.

L'invention a donc pour but de fournir de nouveaux dérivés de purine possédant notamment des propriétés anti-prolifératives.

L'invention vise également un procédé d'obtention de ces dérivés par voie de synthèse, permettant de les préparer à l'échelle industrielle.

Elle vise également leur application en thérapeutique et en tant qu'herbicide.

Les dérivés de purine de l'invention sont caractérisés en ce qu'ils répondent à la formule I 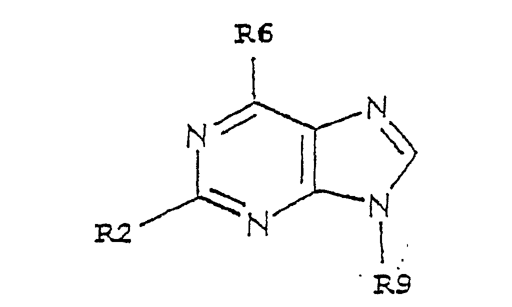 dans laquelle
- R2 représente un radical hydroxypropylamino, 1-hydroxy-2-butylamino, aminoéthylamino, (R)-2-(hydroxyméthyl)pyrrolidin-1-yl, 1-phényl-2-hydroxyéthylamino ou hydroxypentylamino,
- R6 représente un radical benzylamino optionnellement substitué par un halogène, et
- R9 représente un radical isopropyle ou cycloalkyle.

De manière avantageuse, R9 est le groupe isopropyle.

Des dérivés de purine préférés de l'invention sont choisis parmi les composés dans lesquels R2,R6 et R9 sont tels qu'indiqués le tableau 1 suivant :

**TABLEAU 1**

| **R2** | **R6** | **R9** | **IC**_{**50**}**µM cdc2/cycline B** |
|---|---|---|---|
| 3-hydroxypropylamino | benzylamino | isopropyle | 1 |
| 2-hydroxypropylamino | benzylamino | isopropyle | 0,9 |
| 1-D,L-hydroxyméthylpropylamino | benzylamino | isopropyle | 0,65 |
| aminoéthylamino | benzylamino | isopropyle | 1 |
| (2R)-2-hydroxyméthyle-pyrrolidine-1-yl | benzylamino | isopropyle-(9H) | 0,45 |
| (R)-amino-2-phényléthanol | benzylamino | isopropyle-(9H) | 1 |
| (R,S)-amino-pentanol | benzylamino | isopropyle-(9H) | 0,9 |
| (R)-amino-propanol | benzylamino | isopropyle-(9H) | 0,85 |
| (S)-amino-propanol | benzylamino | isopropyle-(9H) | 1 |
| (R)-N-pyrrolidine méthanol | (3-iodo)-benzylamino | isopropyle-(9H) | 0,45µM |
| (R)-N-pyrrolidine méthanol | benzylamino | cyclopentyl(9H) | 0,7 |

Les dérivés suivants sont particulièrement préférés, à savoir : la 2-(R,S)-(6-Benzylamino-9-isopropyl -9 H- purin-2-ylamino)-butan-1-ol, la (2R)-2-hydroxyméthyle-pyrrolidine-1-yl]-6-benzylamino-9-isopropyle-(9H)-purine non cristalline, la 2-(R)-[6-benzylamino-9-isopropyle-(9H)-purine-2-yl]-amino-2-phénylethanol, la 2-(R,S)-[6-benzylamino-9-isopropyle-(9H)-purine-2-yl]-amino-pentanol, la 2-(R)-[6-benzylamino-9-isopropyle-(9H)-purine-2-yl]-amino-propanol, la 2-(S)-[6-benzylamino-9-isopropyle-(9H)-purine-2-yl]-amino-propanol, la 2-(R)-(-)-[6-(3-iodo)-benzylamino-9-isopropyle-(9H)-purine-2-yl]-N-pyrrolidine méthanol et la 2-(R)-(-)-[6-benzylamino-9-cyclopentyle-(9H)-purine-2-yl]-N-pyrrolidine méthanol.

L'invention vise également les isomères optiques et les mélanges racémiques des dérivés définis ci-dessus, en particulier l'isomère R du (2-[6-benzylamino-9-isopropyle-(9H)-purine-2-yl]-amino-2-phényléthanol et du 2-[6-benzylamino-9-isopropyle-(9H)-purine-2-yl]-amino-propanol.

Les dérivés définis ci-dessus sont obtenus selon les méthodes classiques de la synthèse organique. On utilise un dérivé de purine de départ dont les substitutions permettent d'introduire les groupements souhaités.

En utilisant par exemple un dérivé de purine 2-chlore, 6-benzylamino, il est possible d'introduire un groupe alkyle en position 9, par réaction avec par exemple l'halogénure d'alkyle correspondant.

La réaction avec un aminoalcool permettra ensuite d'introduire, en position 2, un groupe alkyl-hydroxyalkylamino à la place du groupe chloro.

Selon un aspect de grand intérêt, les dérivés de l'invention possèdent des propriétés inhibitrices de kinases de grande sélectivité. Ces effets inhibiteurs sont réversibles.

Les cdk jouant un rôle central dans l'initiation, le développement et l'achèvement des événements du cycle cellulaire, les molécules inhibitrices de cdk sont susceptibles de limiter une prolifération cellulaire non désirée telle que cancer, psoriasis, croissance de champignons, de parasites (animaux, protistes) mais aussi de plantes (herbicides) et d'intervenir dans la régulation de maladies neurodégénératives telles que apoptose neuronale et de la maladie d'Alzheimer.

Les kinases plus spécialement sensibles aux effets inhibiteurs de ces dérivés sont les cdc2, les cdk2 et les cdk5.

Leur inhibition est obtenue avec des doses très faibles en dérivés de purine.

Ainsi, on observe le plus généralement une IC₅₀ vis-à-vis de cdc2 inférieure à 50 µM, et même à celle de l'olomoucine (7 µM), considérée cependant comme un inhibiteur puissant.

L'invention vise en particulier des dérivés de purine présentant une IC₅₀ ne dépassant pas 5µM et tout spécialement la 2-(1-D,L-hydroxyméthylpropylamino-)-6-benzylamino-9-isopropylpurine, appelée aussi ci-après roscovitine, dont la IC₅₀ est de 0,65µM, la 6-benzylamino-2-[(2R)-2-hydroxyméthyl-pyrrolidine-1-yl]-9-isopropyle-(9H)-purine non cristalline, le 2-(R)-(-)-[6-(3-iodo)-benzylamino-9-isopropyle-(9H)-purine-2-yl]-N-pyrrolidine méthanol.

Ce dérivé, qui constitue un inhibiteur de grande efficacité et de grande sélectivité vis-à-vis des cdk, cdc2, cdk2 et cdk5 présente en revanche, d'une manière inattendue, des effets sur les kinases erk 1 et erk 2 similaires à ceux de l'olomoucine. La sélectivité est donc nettement supérieure vis-à-vis des kinases cyclines dépendantes. Cet avantage, que l'on retrouve avec les autres dérivés de purine de l'invention, permet d'éliminer les interférences avec les voies de transduction des signaux plus en amont qui impliquent les kinases erk 1 et erk 2 dans de nombreuses réponses cellulaires autres que la division cellulaire.

L'invention vise également les complexes des dérivés de purine avec les cdk et tout spécialement la forme cristallisée du complexe de cdk2 et de roscovitine.

Les études réalisées sur les dérivés de l'invention ont montré, en plus de leurs propriétés 'inhibitrices spécifiques de kinases, des effets cellulaires et sur l'apoptose de grand intérêt.

Ainsi, à très faible concentration (micromolaire pour la roscovitine et bon nombre de dérivés), ils sont capables d'inhiber la transition prophase/métaphase, comme montré par les expériences réalisées sur des ovocytes d'étoile de mer et des embryons d'oursin, et qui sont rapportées dans les exemples.

Sur des extraits acellulaires de Xénope, ils sont capables d'inhiber à la fois le facteur promoteur de la phase M et la synthèse d'ADN.

De manière avantageuse, ces effets cellulaires sont obtenus à des concentrations très faibles en dérivés.

On sait que divers travaux portent sur les rapports existant entre le cycle cellulaire et l'apoptose. Diverses voies conduisent à l'apoptose des cellules dont certaines sont dépendantes de kinases et d'autres ne semblent pas au contraire nécessiter ces enzymes. On a montré que l'apoptose peut être induite au stade G1 ou G2 et qu'à la suite de dommages de l'ADN, certaines cellules s'arrêtent au stade G1 et une voie apoptique dépendante de p53 est alors induite.

Dans d'autres situations, il apparaît que les cellules s'arrêtent au stade G2/M, en réponse à des dommages causés à l'ADN et on observe l'activation d'une voie apoptotique p53 indépendante.

Cette voie se révèle particulièrement importante pour la thérapie de tumeurs dans lesquelles on observe une perte de p53 active.

On mesure donc l'intérêt de disposer, avec les dérivés de l'invention, de moyens pour stimuler une apoptose p53-indépendante dans des cellules arrêtées au stade G2 par endommagement de l'ADN à l'aide d'agents tels que la mitoxantrone ou le cis-platine.

Les inhibiteurs de cdc2 de l'invention peuvent ainsi augmenter les effets thérapeutiques d'agents anti-tumoraux couramment utilisés.

En tant qu'inhibiteurs de cdk5, les dérivés de l'invention peuvent également jouer un rôle pour réduire l'hyperphosphorylation anormale de tau observée durant la maladie d'Alzheimer.

A ces différentes propriétés avantageuses, s'ajoute l'intérêt d'une absence de cyto-toxicité des dérivés de l'invention.

L'invention vise donc la mise à profit des propriétés de ces dérivés en particulier leurs propriétés antimitotiques et anti-neurodégénératives, pour l'élaboration de compositions pharmaceutiques.

Les compositions pharmaceutiques de l'invention sont caractérisées en ce qu'elles renferment une quantité efficace d'au moins un dérivé de purine tel que défini ci-dessus, en association avec un véhicule pharmaceutique inerte.

Les compositions de l'invention sont particulièrement appropriées comme médicaments antimitotiques, en particulier pour la chimiothérapie des cancers, ou encore pour le traitement de psoriasis, de parasitoses telles que celles dues à des protistes ou à des champignons, ou de la maladie d' Alzheimer, ou de l'apoptose neuronale.

Ces compositions renferment le cas échéant des principes actifs d'autres médicaments. On citera notamment leur association avec des médicaments antimitotiques, tels que.ceux à base de taxol, cis-platine, les agents intercalant de l'ADN, et autres.

Les conditionnements en vue de la vente, en particulier l'étiquetage et les notices d'emploi, et avantageusement l'emballage, sont élaborés en fonction de l'application thérapeutique prévue.

Les compositions pharmaceutiques de l'invention sont administrables sous différentes formes, plus spécialement par voie orale ou injectable.

Pour l'administration par voie orale, on a recours en particulier à des comprimés, pilules, tablettes, gélules, gouttes. Ces compositions renferment avantageusement de.1 à 100 mg de principe actif par unité de prise, de préférence de 10 à 40 mg.

D'autres formes d'administration comprennent des solutions injectables par voie intra-veineuse, sous-cutanée ou intra-musculaire, élaborées à partir de solutions stériles ou stérilisables. Il peut s'agir également de suspensions ou d'émulsions.

Ces formes injectables renferment par unité de prise de 1 à 50 mg de principe actif, de préférence de 10 à 30 mg.

A titre indicatif, la posologie utilisable chez l'homme correspond aux doses suivantes : ainsi on administre par exemple au patient en une ou plusieurs prises 10 à 50 mg/jour pour le traitement de tumeurs, ou pour traiter les psoriasis ou des parasitoses.

L'invention vise également des compositions herbicides comprenant au moins un dérivé de purine tel que défini ci-dessus, éventuellement associé à d'autres agents phytopharmaceutiques.

L'invention vise encore les réactifs biologiques dont les principes actifs sont constitués par les dérivés de purine définis plus haut.

Ces réactifs peuvent être utilisés comme références ou étalons dans des études de la division cellulaire.

D'autres caractéristiques et avantages de l'invention sont rapportés dans les exemples qui suivent et en se reportant aux figures 1 à 8, dans lesquelles
- la figure 1, représente les résultats de cinétiques en conditions linéaires à partir d'essais concernant l'activité de la p34^{cdc2}/cycline B à différentes concentrations de roscovitine,
- la figure 2, le pourcentage de rupture de vésicule germinale d'ovocytes d'étoile de mer en fonction de la concentration en roscovitine,
- la figure 3, les effets de la roscovitine sur la maturation d'ovocytes d'étoiles de mer,
- la figure 4, les effets de la roscovitine sur le cycle mitotique d'embryons d'oursins,
- la figure 5, ces embryons arrêtés au stade prophase tardif, et
- la figure 6, les effets de la roscovitine sur la synthèse d'ADN in vitro et l'activité MPF,
- la figure 7 représente les effets de la roscovitine sur l'inhibition de la croissance de cellules L1210 et l'arrêt de leur cycle cellulaire en G2/M, en figure 7A est représentée la croissance de cellules L1210 après deux jours d'exposition à diverses concentrations de roscovitine (moyenne ± déviation standard relative à la croissance de cellule témoin non traitée), en figure 7B sont représentées les moyennes (± déviation standard) de la distribution du cycle de cellules qui ont d'abord été cultivées pendant 48 heures en présence ou en absence de roscovitine 60 µM ,
- la figure 8, représente l'effet d'inhibition de la roscovitine sur la phosphorylation *in vivo* de vimentine au niveau de sites spécifiques à cdc2.

### MATERIEL ET METHODES

### Produits chimiques

Ortho-vanadate de sodium , 1-méthyladénine (1MeAde), EGTA, EDTA, MOPS, β-glycérophosphate, dithiothréitol (DTT), fluorure de sodium, nitrophénylphosphate, leupeptine, aprotinine, inhibiteur de trypsine de soja, benzamidine, histone H1 (type III-S), protéine basique de myéline, caséine, protamine sulfate, isopropyl β-D-thiogalactopyranoside (IPTG), Sépharose 4B activé par CNBr, milieu LB, glutathione et billes de glutathione-Sépharose : tous ces produits sont tels que commercialisés par Sigma Chemicals.

Les analogues de purine sont généralement dissous de manière à disposer de solutions de stockage de 100 mM dans du DMSO. La concentration finale en DMSO dans le mélange réactionnel est inférieure à 1% (v/v).

Le [γ-³²P]-ATP est un produit d'Amersham.

La protéine GST-Rétinoblastome est exprimée dans des bactéries et purifiée sur des billes de glutathione-Sépharose comme décrit précédemment dans (1) et (2).

### Tampons

### Tampon d'homogénéisation :

60 mM de β-glycérophosphate, 15 mM de p-nitrophényl-phosphate, 25 mM de MOPS (pH 7,2), 15 mM d'EGTA, 15 mM de MgCl₂, 1 mM de DTT, 1 mM de vanadate de sodium, 1 mM de NaF, 1 mM de phénylphosphate, 10 µg de leupeptine/ml, 10 µg de aprotinine/ml, 10 µg d'inhibiteur de trypsine de soja ml et 100 µM de benzamidine.

Tampon C: composition du tampon d'homogénéisation, mais avec 5 mM d'EGTA, sans NaF et sans inhibiteurs de protéase.

### Préparation des extraits d'ovocytes d'étoile de mer en phase M

Pour obtenir des préparations d'extraits d'ovocytes à grande échelle, on enlève les gonades de Marthasterias glacialis matures et on les fait incuber avec 10 µM de 1-MeAde dans de l'eau de mer naturelle filtrée sur Millipore jusqu'à la ponte. Les ovocytes sont alors tous entrés en phase M. On les sépare du milieu d'incubation par centrifugation, on les congèle directement dans de l'azote liquide et on les maintient à -80°C (voir (1) et (3)).

Les ovocytes en phase M sont homogénéisés dans le tampon d'homogénéisation à raison de 2 ml/g d'ovocytes.

Après centrifugation pendant 45 minutes à 100000g, le surnageant est récupéré et directement utilisé pour purification par chromatographie d'affinité de la kinase p34^{cdc2} / cycline B sur des billes de p9^{CKShs1}-Sépharose (voir (1) et (4)).

### Enzymes

Les activités des kinases sont déterminées à 30°C dans le tampon C, à moins d'indication contraire. Les valeurs des blancs sont soustraites des données et les activités sont calculées en pmoles de phosphate incorporé dans l'accepteur de protéine pour une incubation de 10 minutes.

Les témoins sont utilisés avec des dilutions appropriées de DMSO.

La phosphorylation du substrat est, le cas échéant, déterminée par autoradiographie après SDS-PAGE.
. On purifie la p34^{cdc2} / cycline B à partir des ovocytes de la phase M des étoiles de mer par chromatographie d'affinité sur les billes de p9^{CKShs1}-Sépharose d'où elles sont éluées à l'aide de p9^{CKShs1} comme décrit ci-dessus (voir (2), (3) et (5)).
   Pour la détermination, on utilise 1 mg d'histone H1 (Sigma type III-S)/ml, en présence de 15 µM de [γ-³²P] ATP (3,000 Ci/mmole, 1 mCi/ml) dans un volume final de 30 µl (voir (1) et (6)).
   Après un temps d'incubation de 10 minutes à 30°C, on dépose des aliquotes de 25 µl de surnageant sur du papier de phosphocellulose Whatman P81 et, après 20 secondes, on lave les filtres 5 fois (pendant au moins 5 minutes à chaque fois) dans une solution de 10 ml d'acide phosphorique par litre d'eau.
   Les filtres humides sont transférés dans des ampoules de scintillation en plastique de 6 ml, puis on ajoute 5 ml de fluide de scintillation ACS (Amersham) et on mesure la radioactivité dans un compteur Packard.
   L'activité kinase est exprimée en pmoles de phosphate incorporé dans i'histone H1 pour 10 minutes d'incubation ou en pourcentage d'activité maximale.
   Pour réaliser les expériences de cinétique en conditions linéaires, on utilise, comme décrit ci-dessus, le système d'essai à point final pour la kinase p34^{cdc2} mais, sur la base d'essais préliminaires, on utilise des concentrations appropriées, non saturantes, en substrat.
   La kinase p34^{cdc2} /cycline B est ajoutée de manière à obtenir une activité linéaire par rapport à la concentration en enzyme et au temps.
   Dans la plupart des cas, ceci nécessite une dilution enzymatique de 3 à 10 fois dans le tampon C.
   Les données de vitesse sont exprimées en pmoles incorporées dans le substrat par seconde, par quantité d'enzyme ajoutée. Les constantes d'inhibition apparentes sont déterminées par analyse graphique.
. Les p33 ^{cdk2}/cycline A et p33 ^{cdk2}/cycline E sont reconstituées à partir d'extraits de cellules d'insectes sf9 infectées par différents baculovirus.
   Les cyclines A et E sont des protéines de fusion de GST-cyclines et les complexes sont purifiés sur des billes de glutathione-Sépharose.
   Les activités kinases sont déterminées avec 1 mg/ml d'histone H1 (Sigma, type IIIS), en présence de 15 µM de [γ-³²P] ATP, pendant 10 minutes, dans un volume final de 30 µl, comme décrit pour la kinase p34^{cdc2}/cycline B.
. La p33 ^{cdk5}/p25 est purifiée à partir de cerveau bovin (7), mais on n'utilise pas l'étape de chromatographie Mono S.
   Les fractions actives récupérées à partir de la colonne de Superose 12 sont réunies et concentrées jusqu'à une concentration finale d'environ 25 µg d'enzyme/ml.
   La détermination de la kinase est effectuée avec 1 mg/ml d'histone H1 (Sigma, type IIIS), en présence de 15 µM de [γ-³²P] ATP, durant 10 minutes dans un volume final de 30 µl, comme décrit pour la p34^{cdc2}/cycline B.
. La p33 ^{cdk4}/cycline D1, est obtenue à partir de lysats de cellules d'insectes. Cdk4 est un produit de construction GST-cdk4 et le complexe actif est purifié sur des billes de glutathione-Sépharose.
   Son activité kinase est déterminée avec une protéine purifiée GST-rétinoblastome en présence de 15 µM de [γ-³²P] ATP, dans un volume final de 30 µl.
   Après 15 minutes d'incubation on ajoute du tampon de Laemmli (2 x 30µl).
   Le substrat phosphorylé est résolu par SDS-PAGE 10% et analysé par autoradiographie par exposition durant environ 14 heures à MP Hyperfilm et densitométrie.
. La p33 ^{cdk6}/cycline D2) est obtenue à partir de lysats de cellules d'insectes (8). Pour les essais, on opère comme indiqué ci-dessus pour la protéine p33 ^{cdk4}/cycline D1.
. Les kinases MAP : La GST-erk1 (9), clonée à partir d'une banque humaine HepG2, est exprimée dans des bactéries, purifiée sur des billes de glutathione-Sépharose et testée avec 1 mg de protéine basique de myéline par ml en présence de 15 µM de [γ-³²P] ATP comme décrit ci-dessus pour la kinase p34^{cdc2}/cycline B.
   Les protéines erk1 et erk2 marquées à l'aide de l'histone sont activées in vitro par MAPKK, purifiées (affinité-Ni et Mono Q) et testées comme décrit ci-dessus durant 10 minutes, dans un volume final de 30 µl.
. La sous-unité catalytique de la kinase cAMP-dépendante. purifiée à partir de coeur de bovin est testée avec 1 mg d'histone H1 par ml, en présence de 15 µM de [γ-³²P] ATP, comme décrit ci-dessus pour la p34^{cdc2}/cycline B.
   La kinase cGMP-dépendante (10), purifiée jusqu'à l'homogénéité à partir de muscle lisse de trachée d'origine bovine est testée avec 1 mg d'histone H1 par ml, en présence de 15 µM de [γ-³²P] ATP, comme décrit ci-dessus pour la p34^{cdc2}/cycline B.
. La caséine kinase 2 est isolée de cytosol de foie de rat (11) et testée avec 1 mg de caséine par ml et 15 µM de [γ-³²P] ATP. Le substrat est déposé sur des filtres Whatman 3MM et lavé avec 10 % de TCA (W/V).
. La kinase de la chaîne légère de myosine purifiée à partir de gésier de poule (12) est testée en présence de 100 nM de calmoduline, 100 µM de CaCl₂, 50mM de HEPES, 5 mM de MgCl₂, 1 mM de DTT et 0,1 mg de BSA/ml à pH 7,5, en utilisant un peptide de synthèse à base du site de phosphorylation de la chaîne légère de myosine de muscle lisse (KKRPQRATSNVFAM, 50 µM) et en présence de 15 µM de [γ-³²P] ATP, dans un volume final de 50 µl.
   L'incorporation de phosphate radioactif est contrôlée sur filtres de phosphocellulose comme décrit ci-dessus.
. L'ASK-γ, homologue chez la plante de GSK-3, est exprimée en tant que protéine de fusion GST dans E. coli (13) et purifiée sur glutathione-Sépharose. L'activité de la kinase ASK-γ est déterminée, pendant 10 minutes à 30°C, avec 5 µgm, de protéine basique de myéline, en présence de 15 µM de [γ-³²P] ATP, dans un volume final de 30 µl. La protéine basique de myéline phosphorylée est récupérée sur papier de phosphocellulose Whatman P81 comme décrit ci-dessus pour la p34^{cdc2}/cycline B.
. Le domaine tyrosine kinasique du récepteur de l'insuline (14) est surexprimé dans un système à baculovirus et purifié jusqu'à l'homogénéité. Son activité kinase est déterminée pendant 10 minutes à 30°C avec 5 µg de Raytide (Oncogène Sciences), en présence de 15 µM de [γ-³²P] ATP, dans un volume final de 30 µl. Le produit Raytide phosphorylé est récupéré sur papier de phosphocellulose Whatman P81 comme décrit ci-dessus pour la p34^{cdc2}/cycline B.

### Exemple 1 : Synthèse de la roscovitine

La synthèse est effectuée en 3 étapes et comporte la préparation 1) tout d'abord de la 6-benzylamino-2-chloropurine, puis 2) de la 6-benzylamino-2-chloro-9-isopropylpurine, et 3) de la 6-benzylamino-2-R-(1-éthyl, 2-hydroxyéthylamino)-9-isopropylpurine.

### 1) Synthèse de la 6-benzylamino-2-chloropurine :

On opère comme décrit par Hocart dans Phytochemistry 1991, 30, 2477-2486.

### 2) Synthèse de la 6-benzylamino-2-chloro-9-isopropylpurine (I) :

On soumet à agitation à température ambiante, pendant trois jours, un mélange de 6-benzylamino-2-chloropurine (3,7 g; 14,2mmole), de carbonate de potassium (llg; 80/mmole) et de bromure d'isopropyle (8,2 ml; 87mmole) dans 100 ml de DMSO absolu. Par chromatographie en couche mince [CHCl₃-MeOH (98:2)], on constate l'absence de 6-benzylamino-2-chloropurine. On élimine par distillation sous vide en-dessous de 50°C le DMSO et l'excès de bromure d'isopropyle. Le résidu est partagé entre de l'eau et de l'acétate d'éthyle. La phase organique est séchée sur Na₂SO₄ et évaporée sous vide.

Par cristallisation dans MeOH, on obtient 3,51 g (82%) de produit ; P.F. 181-182°C; UV (MeOH) : λₘₐₓ 273,5;IR (Nicolet 205, KBr, DRIFT cm 1713,1626,1572,1537, 1497, 1471,1456,1425,1398,1355,1314,1292,1255,1228, 1202.

### 3) Synthèse de la 6-benzylamino-2-R-(1-éthyl-2-hydroxyéthylamino)-9-isopropylpurine (II):

On chauffe dans un four à 160-165°C, pendant 3 h 30, une ampoule scellée, dans laquelle on a fait le vide, contenant 2,7 g (8,95 mmole) de I et 17 ml (0,18 mole) de R(-)-2-amino-1-butanol (Fluka 90%, R:S>9:1). On évapore l'excès d'amine à une température inférieure à 50°C, et on purifie le produit II sur une colonne de chromatographie en utilisant des quantités croissantes de MeOH dans CHCl₃, à savoir 0, puis 2, et 3%.

Par cristallisation dans de l'acétate d'éthyle, on obtient de 2,2 g de II (69%); P.F. 132-134°C, [α] = + 35,1 (c = 0,29, CHCl₃). Spectographie de masse [Finnigam MAT 90, BE géométrie 70 eV, température de la source 250°C, courant d'émission 1 mA, voltage d'accélération 5 keV, entrée directe, DIP température entre 190-220°C. HRMS a été effectué par la méthode de chevauchement de pics en utilisant Ultramark 1600 F (PCR Inc.; Fl.; USA) comme standard] 354.2167 (M⁺, C₁₉H₂₆N₆O calc. 354.2168, 27%), 325 (7%), 324 (29%), 232 (100%), 295 (3%), 282 (7%), 281 (3%), 217 (6%), 185 (5%) 134 (3%), 91 (34%). FTIR (Nicolet 205, KBr, DRIFT, cm⁻¹): 1622,1610,1547,1540,1452,1389, 1370, 1261, 1068.

### Exemple 2 : Etude des propriétés inhibitrices de kinases de la roscovitine et de ses effets sur le cycle cellulaire.

### a) étude des propriétés inhibitrices de kinases.

Les activités des enzymes rapportées dans le tableau suivant ont été mesurées, après addition de roscovitine ou d'olomoucine, selon des concentrations croissantes. Ces activités ont été mesurées avec des substrats appropriés (histone H1, protéine basique de myéline, caséine, etc...) avec 15 µM d'ATP.

Les IC₅₀ ont été calculées à partir des courbes doses-réponses obtenues. L'indication (--) signifie qu'aucun effet inhibiteur n'a été observé. La concentration la plus élevée testée est donnée entre parenthèses.

**TABLEAU 2**

| **Enzyme** | **IC**_{**50**} **(µM) Roscovitine** | **Olomoucine** |
|---|---|---|
| cdc2/cyclin B | 0,65 | 7 |
| cdk2/cyclin A | 0,7 | 7 |
| cdk2/cyclin E | 0,7 | 7 |
| cdk4/cyclin D1 | > 1000 | > 1000 |
| cdk5/p35 | 0,16 | 3 |
| cdk6/cyclin D3 | > 500 | > 250 |
| GST-erk1 | 30 | 30 |
| erk1 | 34 | 50 |
| erk2 | 14 | 40 |
| PK cAMP-dependant | > 1000 | > 2000 |
| PK cGMP-dependant | - (1000) | > 2000 |
| Kinase de la chaîne légère de myosine | 90 | > 1000 |
| caséine kinase 2 | - (1000) | > 2000 |
| ASK-γ (plante GSK-3) | 220 | 130 |
| insuline-récepteur tyrosine kinase | 70 | 400 |
| c-src | 250 | - |
| v-abl | > 1000 | - |

L'examen de ces résultats montre que la roscovitine présente une activité 10 fois plus élevée que l'olomoucine vis-à-vis des cibles cdc2 et cdk2 et 20 fois plus élevée vis-à-vis de cdk5.

Comparativement, son effet apparaît limité, comme observé avec l'olomoucine sur les kinases cdk4/cycline Dlet cdk6/cycline D2 (les IC₅₀ sont supérieures à 100 µM). Cette absence d'effet a été confirmée avec cdk4 provenant de différentes sources. En opérant dans des conditions identiques, la GST-p16^{INK4A} inhibe la cdk4/cycline D1.

### · spécificité de l'effet inhibiteur

Comme on peut le constater, la plupart des kinases sont faiblement ou pas du tout inhibées.

Bien que la roscovitine présente une efficacité au moins 10 fois supérieure à celle de 1' olomoucine vis-à-vis de ses cibles cdk, son effet inhibiteur est très similaire à celui de l'olomoucine vis-à-vis de erk 1 et de erk 2. Une concentration 40 fois plus élevée en roscovitine apparaît ainsi nécessaire pour inhiber erk1 (20 fois pour erk 2) de manière similaire à l'inhibition de cdc2.

### - b) Effet sur l'ATP

Pour étudier le mécanisme d'action de la roscovitine, on a réalisé des expériences de cinétique en présence de concentrations croissantes en roscovitine, avec variation des niveaux de l'ATP (de 0,1 à 0,5 mM), la concentration en histone H1 est maintenue constante à 0,7 mg/ml.

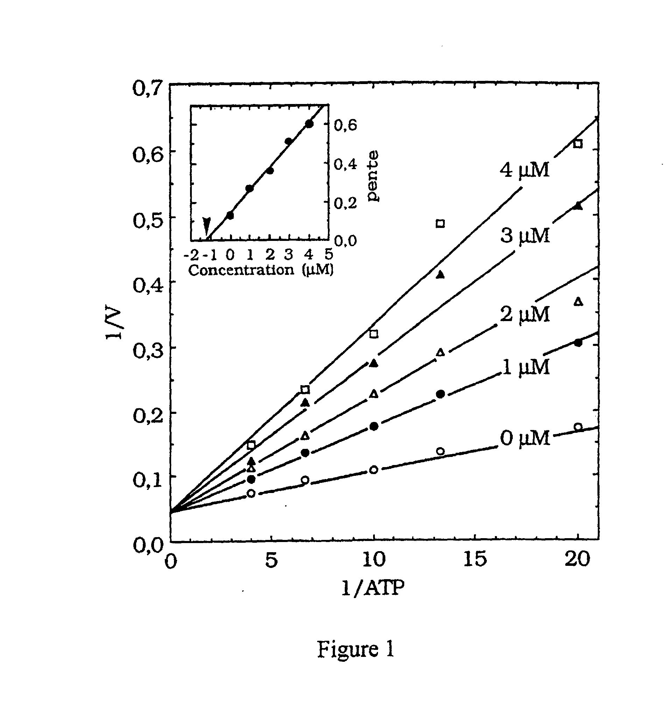

Les résultats sont rapportés sur la figure 1.

Ces résultats montrent que la roscovitine agit en tant qu'inhibiteur compétitif pour l'ATP. Compte tenu de la linéarité des pentes en fonction des concentrations en roscovitine, on la qualifie d'inhibiteur linéaire. La constante d'inhibition apparente Ki est de 1,2 µM.

L'analyse de la structure du co-cristal de roscovitine et de cdk2 confirme que la roscovitine se lie, comme l'olomoucine, dans la poche de liaison à l'ATP et que son cycle purine est orienté comme celui de l'olomoucine, à savoir de manière totalement différente du cycle purine de l'ATP.

### . c) Etude de l'effet sur la synthèse d'ADN et l'activité MPF.

On rapporte les résultats d'expériences réalisées sur plusieurs types cellulaires.

### · effet sur la maturation d'ovocytes d'étoile de mer et sur la déphosphorylation de la tyrosine de p34^{cdc2} in vivo.

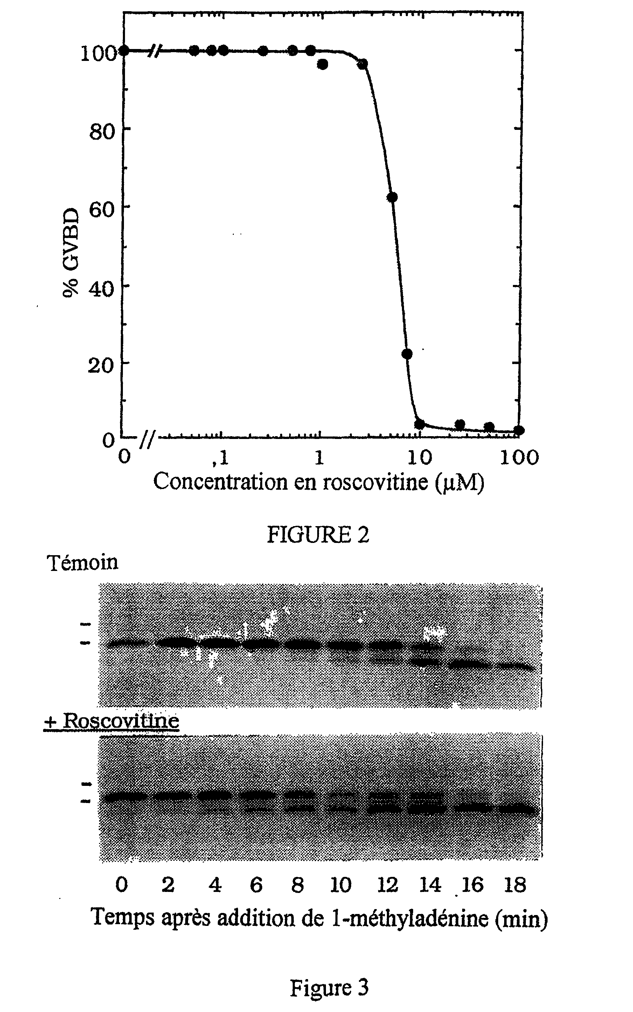

Des ovocytes d'étoiles de mer, arrêtés à la prophase, sont traités, pendant 15 minutes, avec des concentrations croissantes de roscovitine avant d'ajouter l'hormone 1-MeAde (1 µM). Après 30 minutes, on note le % de rupture de vésicule germinale (GVBD). Ces valeurs sont rapportées sur la figure 2 en fonction de la concentration en roscovitine (en µM). La roscovitine inhibe la rupture de l'enveloppe nucléaire avec une IC₅₀ de 5 µM (la IC₅₀ de l'olomoucine, en opérant dans les mêmes conditions, est de 30 µM). Ces résultats sont donnés sur la figure 2.

Comme déjà observé avec l'olomoucine, la roscovitine diminue, mais n'inhibe pas, la déphosphorylation de la tyrosine de p34^{cdc2} in vivo. on traite des ovocytes avec 10 µM de roscovitine pendant 15 minutes avant d'ajouter 1 µM de 1-MeAde au temps 0. Les extraits sont préparés à différents temps et chargés sur une colonne de bille de p9ckshs1-Sépharose

Les protéines qui se sont liées aux billes sont résolues par SDS-PAGE avant d'effectuer un Western blot avec des anticorps anti-PSTAIRE. Une photo du Western blot est présentée sur la figure 3. Les formes phosphorylées de p34^{cdc2} apparaissent dans la partie supérieure et les formes déphosphorylées dans la partie inférieure.

La roscovitine n'inhibe donc pas l'activation de cdc2, mais son activité. La déphosphorylation de la tyrosine de p34^{cdc2} est catalysée par cdc25 et précède normalement l'activation de la kinase cdc2 à la transition G2/M. De plus, la kinase cdc2 phosphoryle et surractive la phosphotase de cdc25. La roscovitine a donc pu provoquer une interruption au niveau cdc2 kinase, entraînant une diminution de la déphosphorylation.

### · effets sur le cycle mitotique d'embryons d'oursins.

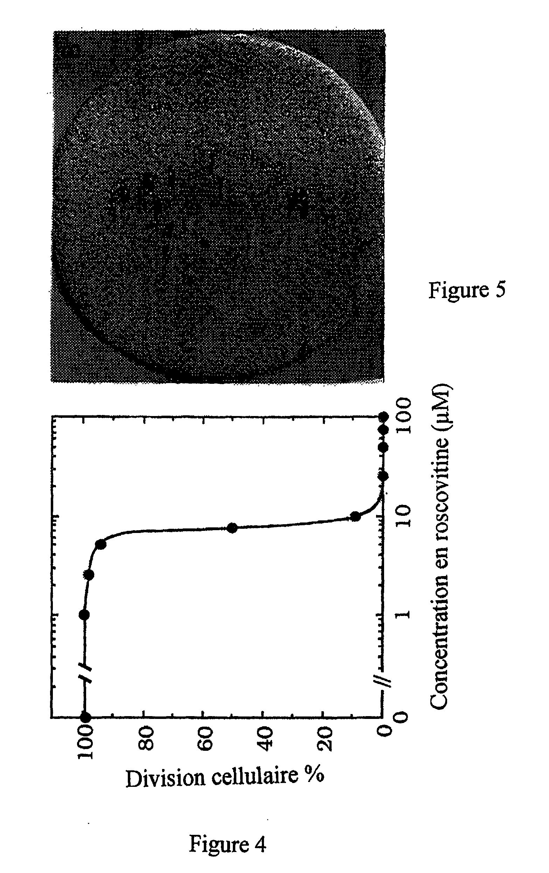

On ajoute de la roscovitine 60 minutes après la fertilisation. Le pourcentage d'embryons divisés est relevé 120 minutes après la fertilisation. Les résultats sont donnés sur la figure 4.

On constate qu'elle provoque un arrêt au stade prophase tardif, qui est dose-dépendant.

La IC₅₀ est de 10 µM. (Même à 100 µM, l'olomoucine ne provoque qu'un ralentissement de la transition préphase/métaphase, mais n'arrête pas les cellules en prophase).

On observe un large noyau dans les oeufs ainsi arrêtés par la roscovitine comme représenté sur la figure 5.

Cet arrêt s'avère totalement réversible. En effet, après plusieurs lavages avec de l'eau de mer, les oeufs entrent à nouveau dans les cycles mitotiques et se développent en larves pluteus normales. Ces résultats sont obtenus même à des concentrations élevées en roscovitine de 100 µM.

### . effets sur la synthèse de l'ADN in vitro et l'activité MPF chez des extraits d'oeufs de Xénope.

Les essais sont réalisés selon (15), en opérant comme décrit dans (1) pour l'olomoucine.

On fait incuber des extraits de Xénope arrêtés au stade métaphase avec de la roscovitine et de la chromatine de sperme.

Avec des concentrations en roscovitine allant de 0 à 5 µM, les chromosomes restent fortement condensés et aucune enveloppe nucléaire n'est visible. A une concentration de 10 µM, et au-dessus, apparaissent des noyaux d'interphase avec de la chromatine partiellement décondensée et une enveloppe nucléaire intacte, montrant que l'activité MPF a été inhibée (la IC₅₀ est de 5 µM).

On a également étudié l'inhibition de la synthèse de l'ADN en opérant comme décrit dans (1) pour l'olomoucine.

On a ainsi ajouté à un extrait d'oeufs, arrêtés au stade métaphase, de la roscovitine et de la chromatine de sperme.

L'extrait a ensuite été abandonné au stade interphase par addition de CaCl₂ (15) et (16). La synthèse de l'ADN total a été mesurée 3 h plus tard par incorporation de [γ-³²P]-dATP dans du matériel précipitable par le TCA.

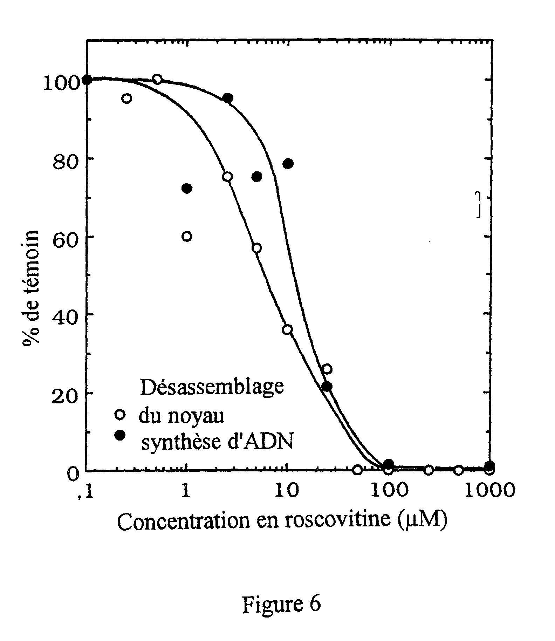

Comme le montre la figure 6, la réplication est inhibée par la roscovitine avec une IC₅₀ de 15 µM.

L'invention fournit ainsi de nouvelles purines dotées de propriétés inhibitrices de cdc2/cycline B de spécificité élevée.

### Exemple 3 : Propriétés biochimiques et effets de la roscovitine sur des cellules de mammifères

### Méthode

### Criblage in vitro de cellules tumorales humaines

Soixante lignées cellulaires de tumeurs humaines comprenant neuf types de tumeurs ont été cultivées pendant 24 heures préalablement à une exposition continue de 48 heures à 0,01-100 µM de roscovitine. Pour estimer la cytotoxicité, un test à la protéine sulforhodaminine B a été utilisée.

### Culture de cellule L1210

Des cellules L1210 prélevées de cultures en croissance exponentielle sur milieu RPMI-1640 supplémenté de sérum de veau foetal à 10%, de pénicilline et de streptomycine ont été comptées à l'aide d'un hémocytomètre, placées à un taux de 5x10⁴ cellules par millilitres dans des plaques de cultures tissulaires à 96 puits en présence ou en absence de diverses concentrations de roscovitine ou d'olomoucine, puis mises à incuber à 37°C sous CO₂ à 5%. Pour inverser l'effet roscovitine, les cellules L1210 cultivées pendant deux jours en présence ou en absence de roscovitine ont été lavées dans du PBS pour éliminer toute trace du produit actif, comptées et replacées dans du milieu frais ne contenant aucun produit actif (roscovitine ou olomoucine). La croissance cellulaire a été mesurée quotidiennement en utilisant le test au tétrazolium sur microculture. L'analyse du cycle cellulaire a été réalisée sur des cellules fixées dans l'éthanol, traitées à la RNase 100 µg/ml et colorées à iodide de propidium. L'acquisition de données a été réalisée à l'aide d'un cytomètre par flux Coulter (Hialeah, Fl, USA) EPICS Elite (marque déposée) et l'analyse de ces données à l'aide d'un logiciel Multicycle (Phoenix Flow Systems, San Diego, CA, USA) (marque déposée). Tous les tests ont été réalisés sur trois répétitions et toutes les expériences ont été répétées au moins deux fois.

### Phosphorylation in vivo de vimentine

Pour étudier la phosphorylation *in vivo* de la vimentine par la kinase cdc2, les cellules ont été soit traitées soit non traitées avec de la roscovitine 60 µM pendant 48 heures préalablement à une exposition de la colcémide 10 ng/ml pendant 2 heures supplémentaires. Les extraits cellulaires ont alors été placés pour migration sur un gel SDS-PAGE 10%, transférés par Western blots et incubés avec des anticorps 4A4. Ces anticorps réagissent de manière croisée avec la vimentine phosphorylée par cdc2 mais ne réagissent ni avec la vimentine phosphorylée par d'autres kinases (protéine kinase cAMP dépendante, protéine kinase C, protéine kinase Ca²⁺-calmoduline-dépendante) ni avec la vimentine non phosphorylée : les anticorps 4A4 reconnaissent spécifiquement la vimentine qui est phosphorylée au niveau de son résidu Ser-55 par la kinase cdc2 lors de l'entrée en mitose de la cellule.

### Résultats

La roscovitine (0,01-100 µM; exposition pendant 48 heures) a été testée sur 60 lignées cellulaires humaines tumorales comprenant neuf types de tumeurs (leucémie, cancer des cellules non-petites de poumons, cancer du colon, cancer du système nerveux central, mélanome, cancer des ovaires, cancer du rein, cancer de la prostate et cancer du sein). Toutes les lignées cellulaires ont présentées une sensibilité équivalente à la roscovitine. La valeur moyenne IC₅₀ est de 16 µM (alors qu'elle est de 60, 3 µM pour l'olomoucine). Aucune corrélation n'a été observée entre la sensibilité des lignées cellulaires à la roscovitine et la présence de p53 sauvage ou muté. La méthode d'analyse Compare a montré que les effets de la roscovitine et du flavopiridol sont comparables.

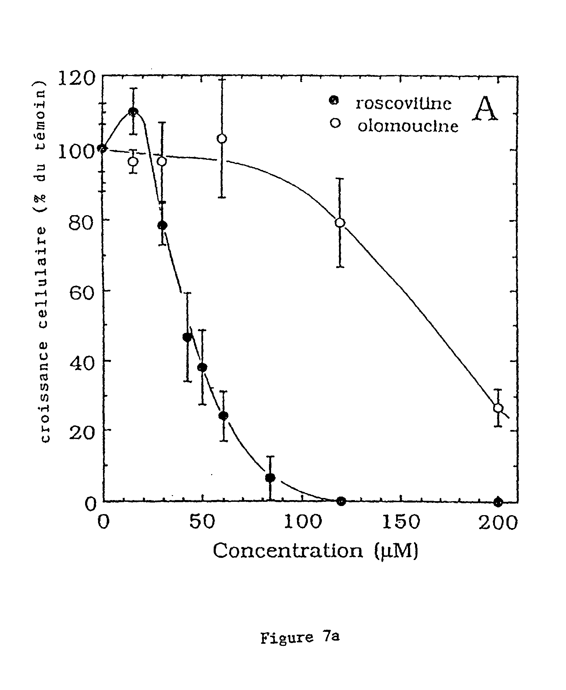

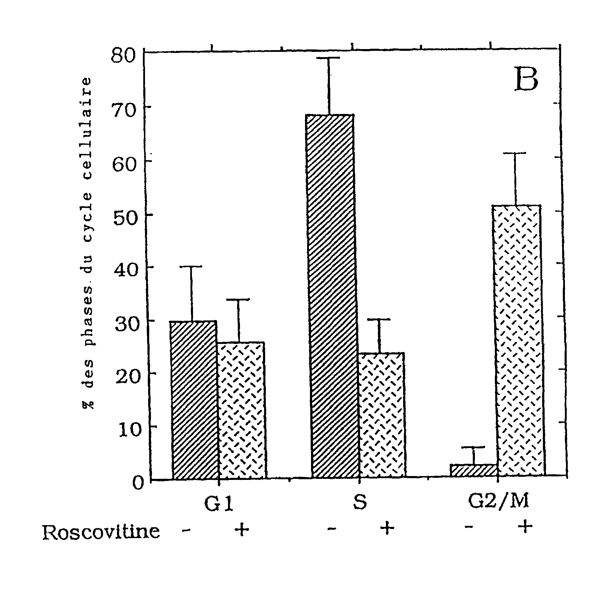

Concernant les effets de la roscovitine sur la croissance de la lignée cellulaire L1210, une inhibition dose-dépendante très nette de la croissance a été observée, comme la montre la figure 7A. La figure 7A où est représentée la croissance cellulaire en fonction de la concentration en roscovitine ou en olomoucine. Les courbes sont sensiblement identiques après deux et trois jours de culture comme observé avec les cellules tumorales ci-dessus mentionnées. La roscovitine est approximativement quatre fois plus efficace que l'olomoucine pour inhiber la croissance cellulaire (IC₅₀ de 40 µM pour la roscovitine et 160 µM pour l'olomoucine). Bien que la plupart des cellules soient viables (96 ± 2 % par exclusion au bleu de Trypan) après un traitement de 48 heures à la roscovitine 60 µM, elles restent irréversiblement stoppées, même après des lavages extensifs. Les cellules exposées à 120 µM de roscovitine meurent rapidement. Les effets de la roscovitine sur la distribution du cycle cellulaire ont ensuite été étudiés par cytométrie de flux. A 60 µM de roscovitine, les cellules restent stoppées en G1 et s'accumulent en G2 comme le montre la figure 7B où sont représentées les proportions (%) de chaque phase du cycle cellulaire observée (G1, S, G2/M) en présence ou en absence de roscovitine.

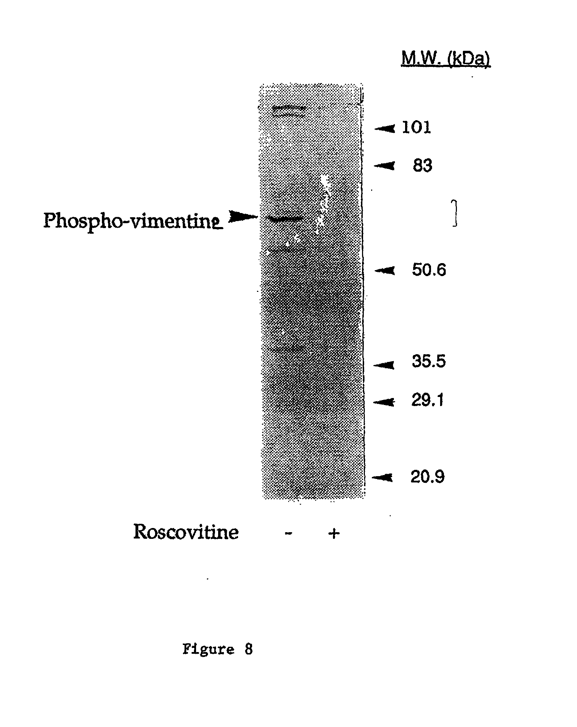

Dans le but d'identifier la cible moléculaire *in vivo* de la roscovitine, des anticorps 4A4 ont été utilisés. Les résultats sont illustrés en figure 8 ou sont représentées les protéines totales extraites des cellules traitées (+) ou non traitées (-) à la roscovitine puis résolues sur SDS-PAGE avant transfert de Western avec les anticorps 4A4. Les cellules non traitées s'arrêtent en métaphase et accumulent de la vimentine phosphorylée par cdc2. Les cellules traitées à la roscovitine ne présentent par contre pas de vimentine phosphorylée par cdc2, ce qui montre que cdc2 a en fait été inhibée *in vivo* et que les cellules ont été stoppées avant la métaphase.

La roscovitine contribue également à réduire l'hyperphosphorylation de tau observée durant la maladie d'Alzheimer : une cdk spécifique du cerveau (cdk5/p35) phosphorylant certains sites de tau est particulièrement sensible à la roscovitine.

### Références Bibliographiques

1 - Vesely et al ((1994) *Eur. J. Biochem*. 224, 771-786.11.
2 - Azzi et al (1994) *J. Biol. Chem. 269*, 13279-13288.
3 - Baratte et al (1992) *Anticancer Res*. 12, 873-880.
4 - Azzi et al (1992) *Eur. J. Biochem*. 203, 353-360.
5 - Richardson et al (1990) *Genes and Developments* 4, 1332- 1344.
6 - Meijer et al (1991) *EMBO J.* 10, 1545-1554.
7 - Lew et al (1992) *J. Biol.* Chem. 267, 13383-13390.
8 - Meyerson et al (1994) *Molecul. Cellul. Biol.* 14, 2077-2086.
9 - Charest et al (1993) *Molecul. Cellul. Biol.* 13, 4679-4690.
10 - Landgraf et al (1989) *Eur. J. Biochem.* 181, 643-650.
11 - Pinna, L.A. (1990) *Biochim. Biophys. Acta* 1054, 267-284.
12 - Craig et al (1987) J. Biol. Chem. 262, 3278--3284.
13 - Blanchi et al (1994) Mol. Gen. Genet. 242, 337-345.
14 - Kallen et al (1990) Biochem. Biophys. Res. Comm. 168, 616-624.
15. Blow et al (1986), Cell 47, 577-587
16. Blow (1993) J. Cell-Biol. 122, 993-1002

## Revendications

1. Dérivés de purine biologiquement actifs, **caractérisés en ce qu'**ils répondent à la formule: 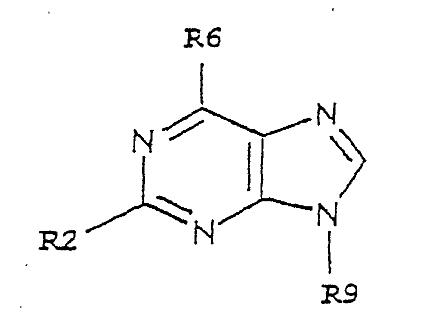 dans laquelle
• R2 représente un radical hydroxypropylamino, 1-hydroxy-2-butylamino, aminoéthylamino, (R)-2-(hydroxyméthyl)pyrrolidin-1-yl, 1-phényl-2-hydroxyéthylamino ou hydroxypentylamino,
• R6 représente un radical benzylamino optionnellement substitué par un halogène, et
• R9 représente un radical isopropyle ou cycloalkyle.

2. Les isomères optiques et les mélanges racémiques des dérivés selon la revendication 1.

3. Le [(R)-1-(6-benzylamino-9-cyclopentyl-9H-purin-2-yl)pyrrolidin-2-yl]méthanol selon la revendication 1 ou 2.

4. Le 2-(R,S) -(6-Benzylamino-9-isopropyl-*9H*-purin-2-ylamino)-butan-1-ol selon la revendication 1 ou 2.

5. Le 2-(R) -(6-Benzylamino-9-isopropyl-*9H*-purin-2-ylamino)-butan-1-ol selon la revendication 1 ou 2.

6. Compositions pharmaceutiques, **caractérisées en ce qu'**elles comprennent une quantité efficace d'au moins un dérivé de purine selon l'une des revendications 1 à 5, en association avec un véhicule pharmaceutique.

7. Compositions pharmaceutiques selon la revendication 6, **caractérisées en ce qu'**elles sont administrables par voie orale ou injectable.

8. Compositions pharmaceutiques selon la revendication 6, **caractérisées en ce qu'**il s'agit de tablettes, comprimés, gélules, pilules et qu'elles renferment de 1 à 100 mg de principe actif par unité de prise, de préférence de 10 à 40 mg.

9. Compositions pharmaceutiques selon la revendication 6, **caractérisées en ce qu'**il s'agit de solutions injectables, ces solutions renfermant avantageusement, par unité de prise, de 1 à 50 mg de principe actif, de préférence de 10 à 30 mg.

10. Compositions pharmaceutiques selon l'une des revendications 6 à 9, **caractérisées en ce qu'**elles sont utilisées comme médicaments antimitotiques.

11. Compositions pharmaceutiques selon la revendication 10, **caractérisées en ce qu'**elles sont utilisées pour la chimiothérapie des cancers.

12. Compositions pharmaceutiques selon l'une quelconque des revendications 6 à 9, **caractérisées en ce qu'**elles sont utilisées pour le traitement de psoriasis.

13. Compositions pharmaceutiques selon l'une quelconque des revendications 6 à 9, **caractérisées en ce qu'**elles sont utilisées pour le traitement de parasitoses.

14. Compositions pharmaceutiques selon la revendication 13, **caractérisées en ce qu'**elles sont utilisées pour le traitement de parasitoses dues à des champignons ou à des protistes.

15. Compositions pharmaceutiques selon l'une quelconque des revendications 6 à 9, **caractérisées en ce qu'**elles sont utilisées pour le traitement de la maladie d'Alzheimer.

16. Compositions pharmaceutiques selon l'une des revendications 6 à 9, **caractérisées en ce qu'**elles sont utilisées comme médicaments antineurodégénératifs, notamment anti-apoptose neuronale.

17. Compositions herbicides, **caractérisés en ce qu'**elles comprennent au moins un dérivé de purine selon l'une des revendications 1 à 5, éventuellement en association avec d'autres agents phytopharmaceutiques.

## Patentansprüche

1. Biologisch aktive Purinderivate, **dadurch gekennzeichnet, daß** sie der folgenden Formel entsprechen: 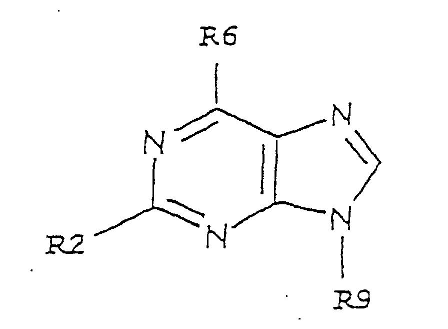 worin
- R2 einen Hydroxypropylamino-, 1-Hydroxy-2-butylamino-, Aminoethylamino-, (R)-2-(Hydroxymethyl)pyrrolidin-1-yl-, 1-Phenyl-2-hydroxyethylamino- oder Hydroxypentylamino-Rest bedeutet,
- R6 einen Benzylamino-Rest bedeutet, der gegebenenfalls durch ein Halogen substituiert ist, und
- R9 einen Isopropyl- oder Cycloalkyl-Rest bezeichnet.

2. Die optischen Isomeren und racemischen Gemische von Derivaten nach Anspruch 1.

3. Das [(R)-1-(6-Benzylamino-9-cyclopentyl-9H-purin-2-yl)pyrrolidin-2-yl]methanol nach Anspruch 1 oder 2.

4. Das 2-(R,S)-(6-Benzylamino-9-isopropyl-9H-purin-2-ylamino)-butan-1-ol nach Anspruch 1 oder 2.

5. Das 2-(R)-(6-Benzylamino-9-isopropyl-9H-purin-2-ylamino)-butan-1-ol nach Anspruch 1 oder 2.

6. Pharmazeutische Zusammensetzungen, **dadurch gekennzeichnet, daß** sie eine wirksame Menge mindestens eines Purinderivats nach einem der Ansprüche 1 bis 5 zusammen mit einem pharmazeutischen Träger enthalten.

7. Pharmazeutische Zusammensetzungen nach Anspruch 6, **dadurch gekennzeichnet, daß** sie oral oder injizierbar verabreichbar sind.

8. Pharmazeutische Zusammensetzungen nach Anspruch 6, **dadurch gekennzeichnet, daß** es sich um Tabletten, Pastillen, Gelatinekapseln, Pillen handelt, und daß sie 1 bis 100 mg, vorzugsweise 10 bis 40 mg des aktiven Prinzips pro Dosis umschließen.

9. Pharmazeutische Zusammensetzungen nach Anspruch 6, **dadurch gekennzeichnet, daß** es sich um injizierbare Lösungen handelt, welche vorteilhafterweise pro Verabreichungseinheit 1 bis 50 mg, vorzugsweise 10 bis 30 mg des aktiven Prinzips enthalten.

10. Pharmazeutische Zusammensetzungen nach einem der Ansprüche 6 bis 9, **dadurch gekennzeichnet, daß** sie als antimitotische Arzneimittel verwendet werden.

11. Pharmazeutische Zusammensetzungen nach Anspruch 10, **dadurch gekennzeichnet, daß** sie für die Krebs-Chemotherapie verwendet werden.

12. Pharmazeutische Zusammensetzungen nach einem der Ansprüche 6 bis 9, **dadurch gekennzeichnet, daß** sie zur Behandlung von Psoriasis verwendet werden.

13. Pharmazeutische Zusammensetzungen nach einem der Ansprüche 6 bis 9, **dadurch gekennzeichnet, daß** sie zur Behandlung von Parasitosen verwendet werden.

14. Pharmazeutische Zusammensetzungen nach Anspruch 13, **dadurch gekennzeichnet, daß** sie zur Behandlung von Parasitosen verwendet werden, welche von Pilzen oder Protozoen verursacht sind.

15. Pharmazeutische Zusammensetzungen nach einem der Ansprüche 6 bis 9, **dadurch gekennzeichnet, daß** sie zur Behandlung der Alzheimer-Krankheit verwendet werden.

16. Pharmazeutische Zusammensetzungen nach einem der Ansprüche 6 bis 9, **dadurch gekennzeichnet, daß** sie als anti-neurodegenerative Medikamente, besonders gegen neuronale Apoptose verwendet werden.

17. Herbizide Zusammensetzungen, **dadurch gekennzeichnet, daß** sie mindestens ein Purinderivat nach einem der Ansprüche 1 bis 5, gegebenenfalls zusammen mit anderen phytopharmazeutischen Mitteln enthalten.

## Claims

1. Biologically active purine derivatives, **characterized in that** they correspond to the formula: 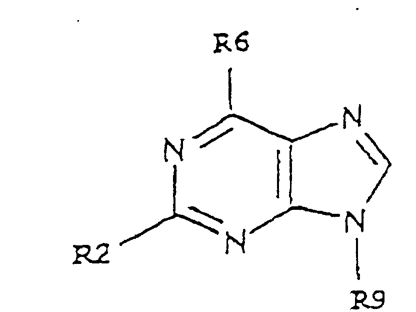 in which
• R2 represents a hydroxypropylamino, 1-hydroxy-2-butylamino, aminoethylamino, (R)-2-(hydroxymethyl)pyrrolidin-1-yl, 1-phenyl-2-hydroxyethylamino or hydroxypentylamino radical
• R6 represents a benzylamino radical optionally substituted by a halogen, and
• R9 represents an isopropyl or cycloalkyl radical.

2. Optical isomers and racemic mixtures of the derivatives according to claim 1.

3. [(R)-1-(6-benzylamino-9-cyclopentyl-9H-purin-2-yl)pyrrolidin-2-yl]methanol according to claim 1 or 2.

4. 2-(R,S)-(6-Benzylamino-9-isopropyl-9H-purin-2-ylamino)-butan-1-ol according to claim 1 or 2.

5. 2-(R)-(6-Benzylamino-9-isopropyl-9H-purin-2-ylamino)-butan-1-ol according to claim 1 or 2.

6. Pharmaceutical compositions, **characterized in that** they comprise an effective quantity of at least one purine derivative according to one of claims 1 to 5 in combination with a pharmaceutical vehicle.

7. Pharmaceutical compositions according to claim 6, **characterized in that** they can be administered by oral or injectable route.

8. Pharmaceutical compositions according to claim 6, **characterized in that** they are tablets, compressed tablets, capsules, pills and that they contain 1 to 100 mg of active ingredient per dosage unit, preferably 10 to 40 mg.

9. Pharmaceutical compositions according to claim 6, **characterized in that** they are injectable solutions, these solutions advantageously containing 1 to 50 mg of active ingredient, preferably 10 to 30 mg, per dosage unit.

10. Pharmaceutical compositions according to one of claims 6 to 9, **characterized in that** they are used as antimitotic medicaments.

11. Pharmaceutical compositions according to claim 10 **characterised in that** they are used for the chemotherapy of cancers.

12. Pharmaceutical compositions according to any one of claims 6 to 9, **characterized in that** they are used for the treatment of psoriasis.

13. Pharmaceutical compositions according to any one of claims 6 to 9, **characterized in that** they are used for the treatment of parasitoses.

14. Pharmaceutical compositions according to claim 13, **characterized in that** they are used for the treatment of parasitoses caused by fungi or protists.

15. Pharmaceutical compositions according to any one of claims 6 to 9, **characterized in that** they are used for the treatment of Alzheimer's disease.

16. Pharmaceutical compositions according to any one of claims 6 to 9, **characterized in that** they are used as antineurodegenerative medicaments, in particular neuronal anti-apoptosis medicaments.

17. Herbicidal compositions, **characterized in that** they comprise at least one purine derivative according to one of claims 1 to 5, optionally in combination with other phytopharmaceutical agents.
